# EUROPEAN PATENT APPLICATION

(11) **EP 3 884 941 A1**
(43) Date of publication of application: **29.09.2021**
(21) Application number: 20382237.4
(22) Date of filing: 26.03.2020
(51) Int. Cl.: A61K 31/426, A61K 31/427, A61P 25/00, A61P 29/00, A61P 31/00, A61P 35/00, A61P 37/00

(54) **INFLAMMASOME INHIBITORS AND USE THEREOF**

(71) Applicant: CENTRO DE INVESTIGACION PRINCIPE FELIPE, 46012 Valencia (ES); Consejo Superior de Investigaciones Científicas (CSIC), 28006 Madrid (ES)
(72) Inventor: ORZÁEZ CALATAYUD, Maria del Mar, 46012 Valencia (ES); SANCHO MEDINA, Mónica, 46012 Valencia (ES); SORIANO TERUEL, Paula, 46012 Valencia (ES); GARCÍA-LAÍNEZ, Guillermo, 46010 Valencia (ES)
(74) Representative: Pons

(57) **Abstract**

The present invention relates to inhibitors of inflammasome of formula (I) and pharmaceutical compositions comprising thereof for use in the treatment of inflammasome-related diseases. These inhibitors disrupt ASC oligomerization and are active in different cellular models of inflammation. Moreover, the compounds inhibit neutrophil infiltration and pro- inflammatory cytokines accumulation in peritonitis *in vivo* model.

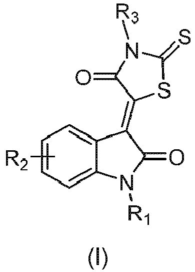

## Description

The invention relates to inhibitors of inflammasome and the use thereof for the treatment of inflammasome-related diseases. Therefore, the present invention belongs to the field of medicine.

### BACKGROUND ART

Inflammasomes are macromolecular complexes from the innate immune system responsible for the maturation and release of pro-inflammatory cytokines. They are formed by a NLR (nucleotide-binding oligomerization domain (NOD) leucine-rich repeat containing receptor) sensor protein, the adaptor protein ASC (the apoptosis-associated speck-like protein containing a caspase activation and recruitment domain (CARD)) and the protease procaspase-1 (PC1) (Martinon F et al. Mol Cell. 2002 Aug;10(2):417-26). Different NLRs recognize specific conserved microbial structures called pathogen-associated molecular patterns (PAMPs) or danger-associated molecular patterns (DAMPs). After recognition, NLRs oligomerize promoting the assembly of ASC, which then binds and activates caspase-1 (C1). The activation of caspase-1 results in the cleavage and secretion of pro-inflammatory cytokines such as IL-1β and IL-18. Caspase-1 also cleaves GSDMD (gasdermin D) which in turn, forms pores in the plasma membrane triggering pyroptotic cell death (Shi J et al. Nature. 2015 Oct 29;526(7575):660-5). ASC interacts through the pyrin domain (PYD) with sensors, and through the CARD with PC1. The formation of ASC oligomers generates a multitude of caspase-1 interaction sites leading to the amplification of the pro-inflammatory signaling (Dick MS et al. Nat Commun. 2016 Jun 22;7:11929). The formation of this ASC- procaspase-1 complexes leads to the proximity-induced activation of PC1. According to the structure of the NLR involved, several types of inflammasomes have been described to date. NLRP1, NLRP3, NLRC4, and AIM2 inflammasomes are the most studied and their ASC dependence is variable. While In the case of NLRP3, and AIM2 the presence of ASC is a requisite for PC1 activation. In the case of CARD containing sensors such as NLRP1 and NLRC4, ASC is not required for initial activation but clearly enhances pro-inflammatory signaling.

The inflammatory signaling engaged by the activation of the inflammasome is a rapid response of the innate immune system to ensure removal of detrimental stimuli and repair damaged tissue. In this sense, inflammasome activation avoids the spread of infections or development of tumors and is necessary in the development of an adaptive immune response (Lamkanfi M and Dixit VM. Annu Rev Cell Dev Biol. 2012;28:137-61). However, inflammasome dysregulation has been implicated in the pathophysiology of several diseases such as Alzheimer's, diabetes or cancer and also leads to many inflammatory and autoimmune disorders including gout, silicosis, rheumatoid arthritis and genetically-inherited periodic fever syndromes (Guo H et al. Nat Med. 2015 Jul; 21(7): 677-687).

With the aim to avoid the consequences of pathological inflammasome activation several strategies have been explored such as targeting the NLRs, caspase-1 direct inhibitors or the antibody blockage of IL-1β or its receptor. Several NLRs inhibitors have been described in the literature. Parthenolide and BAY 11-7082 are broad-spectrum inhibitors with anti-inflammatory activity against multiple targets. MCC950 is the most potent and specific NLRP3 inhibitor that showed efficacy in NLRP3-dependent mouse models although the molecular mechanism of action remains unclear (Coll RC et al. Nat Med. 2015 Mar;21(3):248-55). Recently, a new specific NLRP3 inhibitor, CY-09, was developed. It specifically blocks NLRP3 activation by binding to the ATP-binding motif and showed therapeutic effect on mouse model of cryopirin-associated autoinflammatory syndrome (CAPS) and type 2 diabetes (Jiang H et al. J Exp Med. 2017 Nov 6;214(11):3219-3238). NLRP3 specific inhibitors are of high interest for the treatment of those diseases depending on the activation of this particular inflammasome. However, new approaches are needed to afford multifactorial disorders that with frequency are dependent on the activation of other or more than one inflammasome. This is the case, for example, in the regulation of commensal microbiota where a role of multiple inflammasomes has been described in inflammatory bowel disease. The three NLR proteins (NLRP3, NLRP6 and NLRC4) that have an effect on colitis also influence the incidence of tumor formation (Masters SL. Clin Immunol. 2013 Jun;147(3):223-8). It is also known that there are at least two inflammasomes that can impair metabolism and inflammation to contribute to pathology in type 2 diabetes and other metabolic disorders (Henao-Mejia J et al. Nature. 2012 Feb 9; 482(7384): 179-185). Finally, few years ago two groups described gain-of-function mutations in NLRC4 as the cause of a syndrome of infantile enterocolitis and recurrent Macrophage Activation Syndrome (Canna SW et al. Nat Genet. 2014 Oct;46(10):1140-6; Romberg N et al. Nat Genet. 2014 Oct; 46(10): 1135-1139).

The efforts to block the enzymatic activity of active caspase-1 have failed until now. VX-765 was identified as a small molecule inhibitor of the catalytic site of caspase-1 but clinical trials were discontinued by the lack of effectiveness (Moll M and Kuemmerle-Deschner JB. Clin Immunol. 2013 Jun;147(3):242-75). Similar approaches have been done with pro-apoptotic caspases where the lack of clinical positive outcomes for direct caspase inhibitors has been attributed to the high processivity of these proteases which makes difficult to stop the signaling cascade once is initiated. Alternative strategies, that recently reached clinical trials, are focused in avoiding the activation of caspases. These approximations improve cell death protection and have open new expectations in the apoptosis field (Orzaez M et al. PLoS One. 2014 Oct 20;9(10):e110979).

The only clinical approved treatment for inflammasome-related diseases are the biological agents that target IL-1β, including Anakinra, a recombinant IL-1 receptor antagonist (Granowitz EV et al. Cytokine. 1992 Sep;4(5):353-60), Canakinumab, a neutralizing IL-1β antibody (Rondeau JM et al. MAbs. 2015;7(6):1151-60), and Rilonacept, a soluble decoy IL-1 receptor (Hoffman HM et al. Arthritis Rheum. 2008 Aug;58(8):2443-52). Although effective, the high cost and the side effects reported make the development of new molecules an important challenge for the pharmacological industry. Moreover, they do not address all the consequences of inflammasome activation (such as pyroptotic cell death or IL-18 release).

The present invention proposes new inhibitors of inflammasome based on ASC-PC1 protein-protein interaction as a new point of intervention for modulating inflammasome, down enough in the pathway to avoid side-effects but also upstream enough to stop signaling and pyroptotic cell death.

### SUMMARY OF THE INVENTION

The inventors of the present invention have identified a group of compounds as new modulators of the inflammasome that targets ASC-mediated PC1 activation. They have demonstrated that said compounds disrupt ASC oligomerization and are active in different cellular models of inflammation. Moreover, the compounds inhibit neutrophil infiltration and pro- inflammatory cytokines accumulation in peritonitis *in vivo* model.

Thus, the main objective of the present invention is to provide compounds for use in method of inhibiting inflammasome in a subject suffering from inflammasome-related diseases based on the discovery of new inflammasome inhibitors.

Then, a first aspect of the present invention relates to a compound of formula (I) 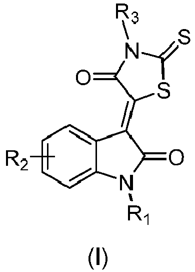
a pharmaceutically acceptable salt, isomers or a solvate thereof, wherein
R₁ is selected form the list comprising: H, optionally substituted (C₁-C₄) alkyl and optionally substituted (C₁-C₄) alkenyl,
R₂ is H or halogen,
R₃ is an optionally substituted (C₁-C₄) alkyl or optionally substituted (C₁-C₄) alkenyl, for use as inflammasome inhibitor.

Preferably, the inhibition takes place in a subject suffering from a inflammasome-related disease. Then, in a preferred embodiment the present invention relates to a compound of formula (I) as described above in the treatment and/or prevention of inflammasome-related diseases.

In a preferred embodiment of the invention, R₁ is H.

In another preferred embodiment of the invention, R₂ is H.

In another preferred embodiment of the invention, R₃ is an optionally substituted (C₁-C₄) alkenyl group. More preferably, R₃ is an optionally substituted C₃ alkenyl group.

In another preferred embodiment of the invention, R₃ is an optionally substituted (C₁-C₄) alkyl group.
In another preferred embodiment of the invention, R₃ is an optionally substituted (C₁-C₂) alkyl group.

If any of the alkyl groups cited above is substituted, it is preferably substituted by a - O(C₁-C₃)alkyl group, by a heterocycle or by a carboxilic acid; it is more preferably substituted by a -O(C₁-C₃)alkyl group or by a heterocycle and even more preferably by a -OCH₃ or by a furanyl.

In another preferred embodiment of the invention, the compound if formula (I) is selected from the next ones:

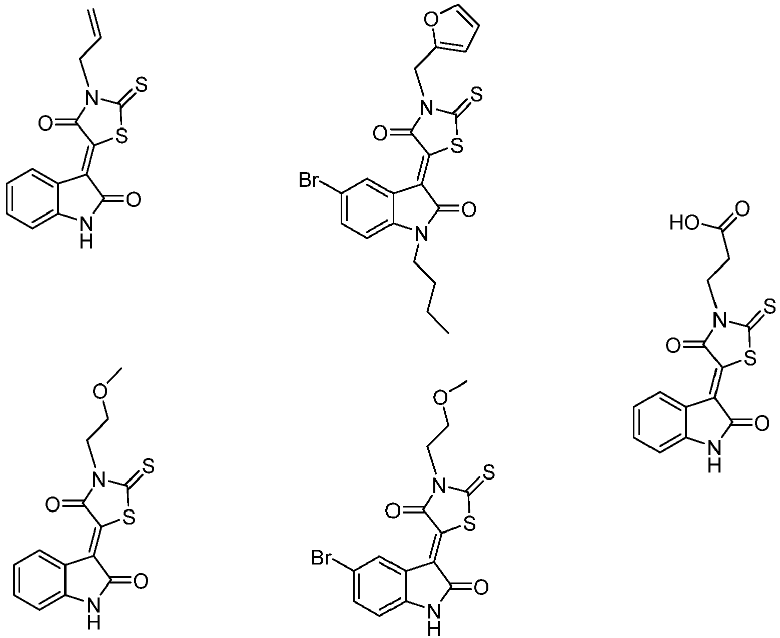

The term "C₁-C₄ alkyl group" in the present invention refers to linear or branched saturated hydrocarbon chain that have 1 to 4 carbon atoms, for example, methyl, ethyl, *n*-propyl, *i*-propyl, *n*-butyl, *sec*-butyl, *tert*-butyl. The C₁-C₄ alkyl groups may be optionally substituted by one or more halogen, hydroxyl, alkoxyl, carbonyl, cyano, acyl, alkoxycarbonyl, amino, nitro, mercapto, alkylthio, heterocycle, carboxylic acid or combinations of these substituents; preferably the substituent is alkoxyl (-O(C₁-C₃)alkyl group) or a heterocycle, such as furane, or a carboxylic acid.

The term "C₁-C₄ alkenyl group" in the present invention refers to linear or branched hydrocarbon chain that have 1 to 4 carbon atoms containing at least one double bond, for example, 1-propenyl, 2-propenyl, 1-butenyl, 2-butenyl, 3-butenyl, 1,3-butadienyl. Such alkenyl moieties may exist in the E or Z configurations; the compounds of this invention include both configurations.

The term "halogen" refers to fluoride, chloride, bromide or iodine.

The term "heterocycle" as used herein, refers to a 3 to 10 membered ring, which can be aromatic or non-aromatic, saturated or unsaturated, containing at least one heteroatom selected form oxygen, nitrogen, sulfur, or combinations of at least two thereof, interrupting the carbocyclic ring structure. In the present invention, the heterocycle preferably contains oxygen.

The term "treatment or prevention" as used herein, unless otherwise indicated, relates to reversing, alleviating and inhibiting the progress of, or preventing the disorder or condition to which it applies in such terms, one or more symptoms of such disorder or condition.

Inflammasome-related diseases refers to a group of diseases in which the inflammasome plays a critical role in the initiation and progress of these diseases. The IL-Iβ and IL-18 induced by the inflammasome play a key role to prime the inflammatory response, since they induce the production of many major inflammatory cytokines (TNFa, IL-6, ...), and to activate various cell types involved in the inflammation (such as macrophages, platelets or neutrophils).

The inflammasome-related diseases include, but are not limited to monogenic inflammasomopathies; infectious and inflammatory diseases, neurodegenerative pathologies and autoimmune diseases.

The monogenic inflammasomopathies are preferably selected from the list comprising: Cryopyrin-Associated Periodic Syndromes (CAPS), Familial Mediterranean Fever (FMF), Schnitzler Syndrome, Hyper-lgD Syndrome (HIDS), PAPA Syndrome and Deficiency of IL-1 Receptor Antagonist (DIRA).

The infectious diseases are preferably selected from the list comprising: Zika, HIV, and West Nile virus, and bacterial infections that induce meningitis.

The autoimmune diseases are preferably selected from the list comprising: lupus erythematosus , topic and contact dermatitis and vitiligo.

The inflammatory diseases are preferably selected from the list comprising: acne, hidradenitis suppurative, psoriasis antinflammatory bowel disease (IBD) such asCrohn's disease (CD), ulcerative colitis (UC) and dysbiosis.

The neurodegenerative pathologies are preferably selected from the list comprising: multiple sclerosis, amyotrophic lateral sclerosis, prion diseases, Parkinson's disease, and Alzheimer's disease, cancer, peritonitis, gout, diabetes and rheumatoid arthritis, atherosclerosis, chronic obstructive pulmonary disease (COPD), hypertension, asthma, allergies, ischemic stroke, ischemia-reperfusion injury and alopecia areata..

In a more preferred embodiment, the inflammasome-related diseases are selected form Cryopyrin-Associated Periodic Syndromes (CAPS), Familial Mediterranean Fever (FMF), Zika, HIV, acne, psoriasis, vitiligo, inflammatory bowel disease (IBD), Crohn's disease (CD), Parkinson's disease, Alzheimer's disease, cancer, peritonitis, gout, diabetes, rheumatoid arthritis, atherosclerosis and chronic obstructive pulmonary disease (COPD).

The compounds of formula (I) may include isomers, depending on the presence of multiple bonds (for example Z, E). Both isomers Z and E are included within the formula (I).

Unless otherwise stated, the compounds of formula (I) are intended to include compounds that differ only in the presence of one or more isotopically enriched atoms. For example, compounds having the present structures except for the substitution of a hydrogen atom for a deuterium atom or a tritium atom, or the substitution of a carbon atom for a carbon atom enriched in ¹³C or ¹⁴C or a nitrogen atom enriched in ¹⁵N fall within the scope of this invention.

It must be understood that compounds of formula (I) encompasses all the isomers of said compounds, i.e. all the geometric, tautomeric and optical forms, and their mixtures (for example, racemic mixtures). When there are more chiral centres in the compounds of formula (I), the present invention includes, within its scope, all the possible diastereomers, including their mixtures. The different isomeric forms may be separated or resolved therebetween using conventional methods or any given isomer can be obtained using conventional synthetic methods or by means of stereospecific, stereoselective or asymmetric synthesis.

The term "pharmaceutically acceptable salts or solvates thereof" relates to salts or solvates which, on being administered to the recipient, are capable of providing a compound such as that described herein. The preparation of salts and derivatives can be carried out by methods known in the state of the art. Preferably, "pharmaceutically acceptable" relates to molecular entities and compositions that are physiologically tolerable and do not typically produce an allergic reaction or a similar unfavourable reaction, such as gastric upset, dizziness and similar side effects, when administered to a human. Preferably, the term "pharmaceutically acceptable" means approved by a regulatory agency of a federal or state government or collected in the US Pharmacopoeia or other generally recognised pharmacopeia for use in animals and, more particularly, in humans.

The compounds of formula (I) may be in crystalline form, either as free compounds or as solvates (e.g.: hydrates), and it is understood that both forms fall within the scope of the present invention. Solvation methods are generally known in the state of the art. Suitable solvates are pharmaceutically acceptable solvates. In a particular embodiment, the solvate is a hydrate.

In a second aspect, the present invention relates to a pharmaceutical composition comprising the compound of formula (I), as described above, a pharmaceutically acceptable salt, isomers or a solvate thereof, for use in the treatment and/or prevention of inflammasome-related diseases.

The pharmaceutical composition preferably includes at least one excipient, adjuvant and/or a pharmaceutically acceptable carrier.

The term "carrier" relates to a dilute, adjuvant or excipient with which the main ingredient is administered. Such pharmaceutical carriers may be sterile liquids, such as water and oils, including those derived from oil, animal, vegetable or synthetic, such as peanut oil, soybean oil, mineral oil, sesame seed oil and similar. Water or aqueous saline solutions and aqueous dextrose and glycerol solutions are preferably used as carriers, particularly for injectable solutions. Preferably, the carriers of the invention are approved by the regulatory agency of a state government or a federal government, or are enumerated in the United States Pharmacopoeia, in the European Pharmacopoeia or other pharmacopoeia recognised in general for use in animals and, more particularly, in humans.

The pharmaceutical composition comprises a therapeutically effective amount of the compound of formula (I), the pharmaceutically acceptable isomers, salts or solvates thereof that must be administered (also referred to herein as therapeutically amount effective thereof).

The therapeutically amount effective of the compound of formula (I), in addition to their dosage for treating a pathological condition with said compound, will depend on a number of factors, including age, the condition of the patient, the severity of the disease, administration route and frequency, the modular compound to be used, etc.

The pharmaceutical compositions of this invention can be used on their own or jointly with other drugs to provide combined therapy. The other drugs can form part of the same pharmaceutical composition or be provided as a separate pharmaceutical composition, to be administered simultaneously or at different intervals. Examples of pharmaceutical compositions include any solid composition (tablets, pills, capsules, granules, etc.) or liquid composition (solutions, suspensions or emulsions) for oral, topical or parenteral administration.

A further aspect of the present invention relates to the use a compound of formula (I) as defined above (including its preferred embodiments), a pharmaceutically acceptable salt or a solvate thereof, as inflammasome inhibitors. More preferably, as inflammasome inhibitors as biotechnology tool for the study of the implication of the inflammasome in biological processes.

In summary, the present invention disclosed new compounds as inflammasome inhibitors that inhibit all the ASC-dependent inflammasomes. This represents an advantage particularly in the case of diseases in which the activation of more than one inflammasome is involved. The point of intervention (ASC-PC1 protein-protein interactions) represents an advantage in terms of cell death inhibition compared with direct caspase-1 inhibitors.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

### DESCRIPTION OF THE DRAWINGS

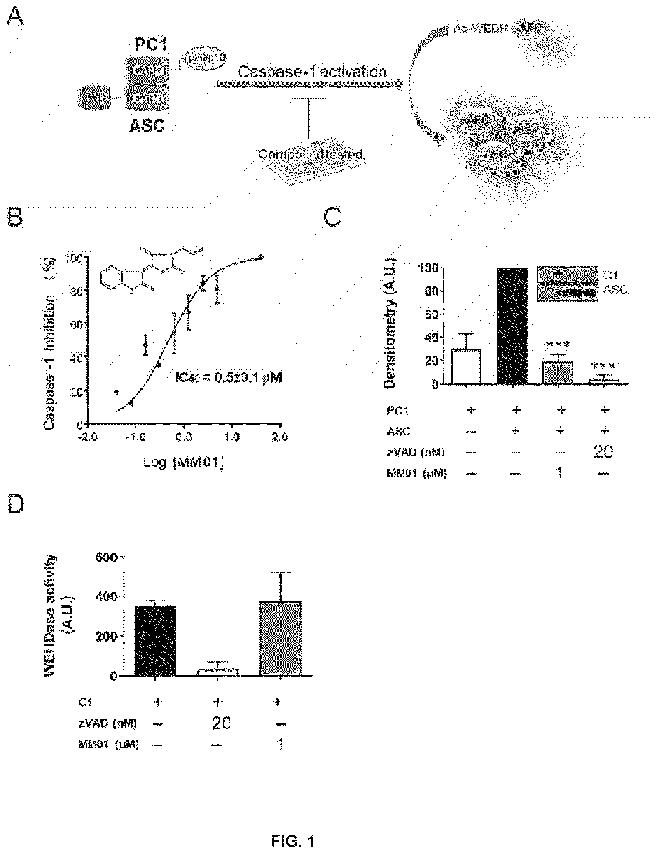

**Fig. 1****.** High throughput screen identifies MM01 as an inhibitor of ASC-mediated PC1 activation. A. Schematic representation of the screening assay. B. Chemical structure and dose-response curve of compound MM01 (0.04, 0.08, 0.16, 0.32, 0.65, 1.25, 2.5, 5, 10, 20 and 40 µM) in the in vitro ASC-mediated PC1 activation assay. Data represent the mean ± SD of three independent experiments. C. Immunoblotting of PC1 processing upon MM01 treatment. An inhibitor of caspase catalytic site (zVAD) was used as control. Bands corresponding to three experiments were quantified and plotted. D. Recombinant caspase-1 activity was monitored in the presence of MM01 in order to exclude compounds affecting active caspase-1. Data represent the mean ± SD of three independent experiments.

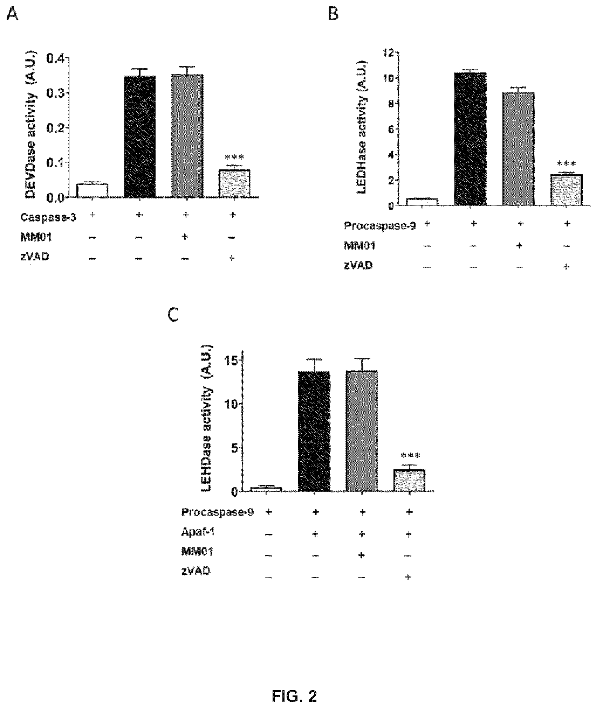

**Fig. 2****.** MM01 inhibits inflammatory PC1 but not apoptotic caspases in vitro. A. Recombinant caspase-3 activity was measured in the presence of zVAD (20 nM) and MM01 (5 µM). B. Recombinant caspase-9 activity was measured in the presence of zVAD (20 nM) and MM01 (5 µM). C. Apoptosome activity was monitored after reconstitution in vitro in the presence of zVAD (20 nM) and MM01 (5 µM).

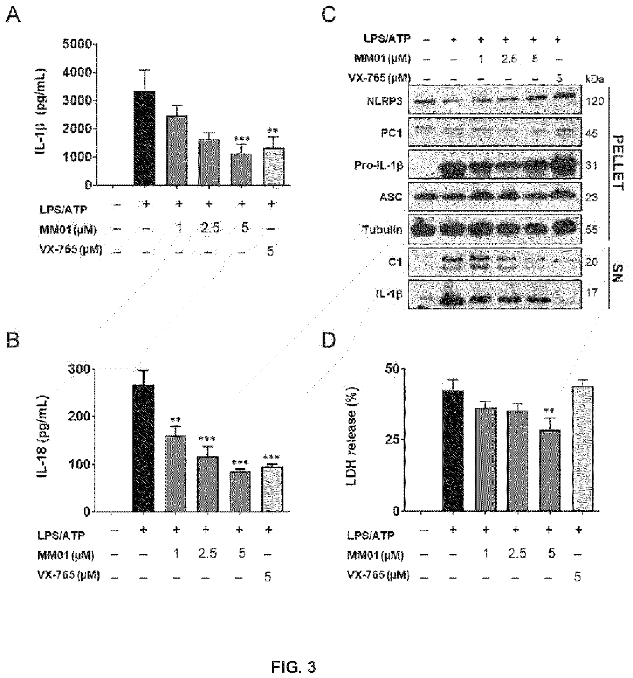

**Fig. 3****.** MM01 inhibits NLRP3 activation mediated by LPS and ATP stimulation in THP-1 cells. IL-1β (A) and IL-18 (B) secretion were evaluated by the ELISA technique upon activation of the NLRP3 inflammasome with LPS (100 ng/ml) and ATP (2.5 mM). Cells were treated with MM01 at 1, 2.5 or 5 µM, and with VX-765 at 5 µM. C. THP-1 cells were stimulated as described above, and supernatants (SN) and pellets were analyzed by immunoblotting; IL-1β and cleaved caspase-1 were detected. D. Measurement of the release of LDH into the extracellular medium under the above-described conditions. Asterisks represent significant differences to the stimulated control (LPS/ATP), as determined by a one-way ANOVA test with Tukey's multiple post-test comparisons **p<0.05; ***p<0.001. All the data are expressed as the mean ± SD of three experiments.

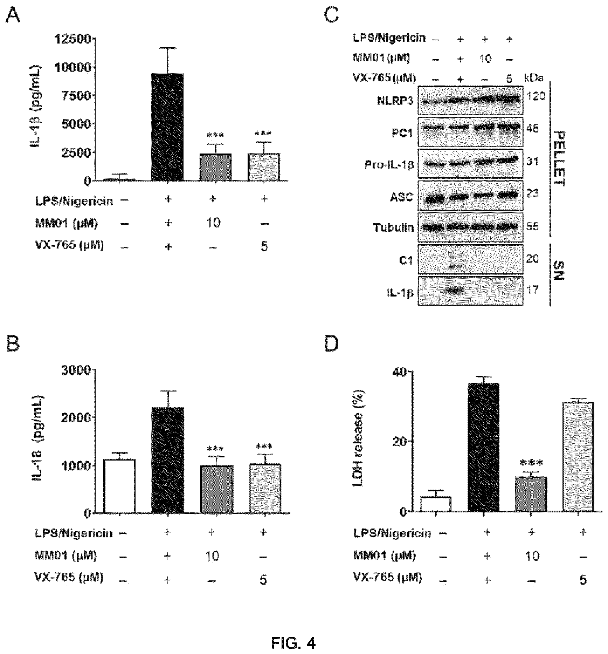

**Fig. 4****.** MM01 inhibits NLRP3 activation mediated by LPS and nigericin stimulation in PMA-differentiated THP-1 cells. IL-1β (A) and IL-18 (B) secretion were evaluated by the ELISA technique upon activation of the NLRP3 inflammasome with LPS (100 ng/ml) and nigericin (10 µM). Cells were treated with MM01 at 10 µM, and with VX-765 at 5 µM. C. THP-1 cells were stimulated as described above, and supernatants (SN) and pellets were analyzed by immunoblotting; IL-1β and cleaved caspase-1 were detected. D. Measurement of the release of LDH into the extracellular medium under the above-described conditions. Asterisks represent significant differences to the stimulated control (LPS/Nigericin), as determined by a one-way ANOVA test with Tukey's multiple post-test comparisons **p<0.05; ***p<0.001. All the data are expressed as the mean ± SD of three experiments.

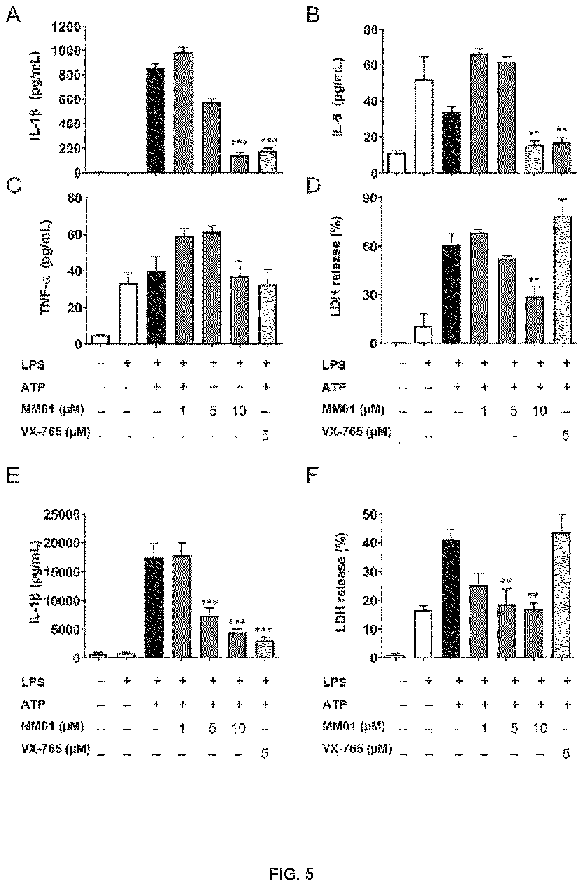

**Fig. 5****.** MM01 inhibits LPS/ATP stimulation of murine peritoneal macrophages and isolated human PBMCs. IL-1β (A), IL-18 (B) and TNF-α (C) secretion was evaluated by the ELISA technique upon activation of the NLRP3 inflammasome with LPS (100 ng/ml) and ATP (2.5 mM) and treated or not with MM01 at 1, 5 or 10 µM, and with VX-765 at 5 µM in isolated peritoneal macrophages. D. Percentage of LDH release under the above-described conditions. Asterisks represent significant differences to the stimulated control (LPS/ATP), as determined by a one-way ANOVA test with Tukey's multiple post-test comparisons **p<0.05; ***p<0.001. All the data are expressed as the mean ± SD of three experiments. IL-1β (E) and LDH (F) release was evaluated upon activation of the NLRP3 inflammasome with LPS (100 ng/ml) and ATP (2.5 mM), treated or not with MM01 at 1, 5 or 10 µM, and with VX-765 at 5 µM in human PBMCs.

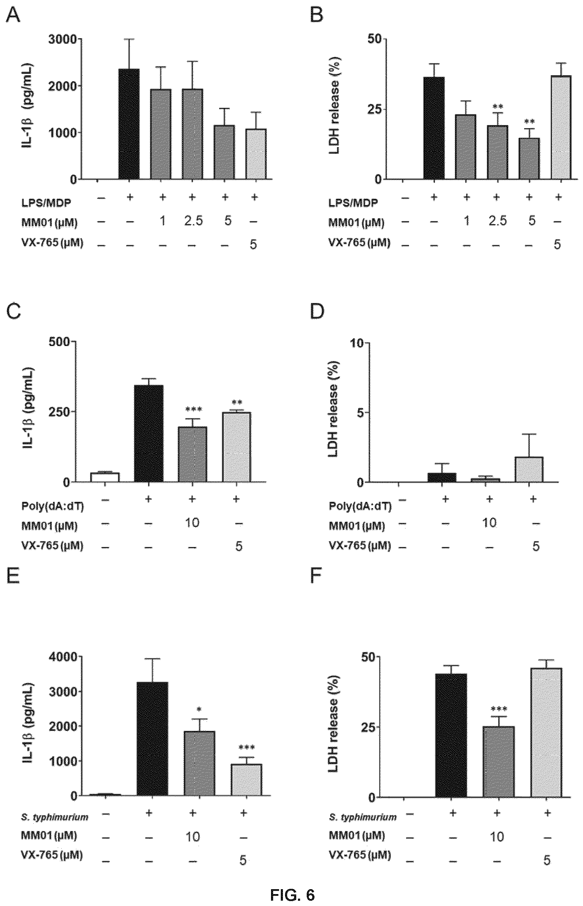

**Fig. 6****.** MM01 inhibits NLRP1, AIM2 and NLRC4 inflammasome activation. IL-1β (A) measured by ELISA and LDH (B) release was evaluated upon activation of the NLRP1 inflammasome with LPS (100 ng/ml) and MDP (50 µg/ml) in THP-1 cells. Cells were treated with MM01 at 1, 2.5 or 5 µM, and with VX-765 at 5 µM. MM01 inhibits AIM2 activation mediated by double-stranded DNA. IL-1β secretion (C) and LDH release (D) were analyzed upon stimulation of the AIM2 inflammasome with poly (dA:dT) (200 µg/ml) in PMA-differentiated THP-1 cells treated or not with MM01 at 10 µM, and with VX-765 at 5 µM. D. Measurement of the release of LDH into the extracellular medium under the above described conditions. MM01 inhibits NLRC4 activation mediated by *Salmonella typhimurium* in THP-1 cells. A. IL-1β (E) and LDH (F) secretion was evaluated upon stimulation. Cells were treated with MM01 at 10 µM, and with VX-765 at 5 µM. Asterisks represent significant differences to the stimulated control as determined by a one-way ANOVA test with Tukey's multiple post-test comparisons **p<0.05; ***p<0.001. All the data are expressed as the mean ± SD of three experiments.

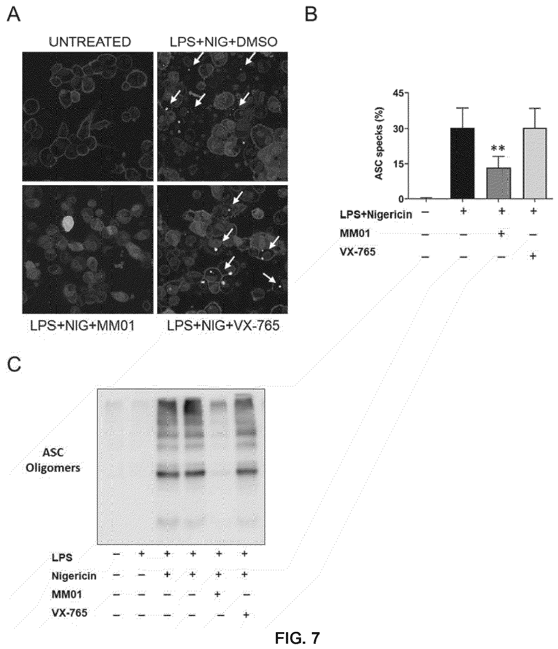

**Fig. 7****.** MM01 inhibits ASC oligomerization. A. Live-cell imaging of THP-1 ASC GFP cells treated with MM01 (10 µM) or VX-765 (5 µM) and stimulated with LPS (100 ng/mL) and nigericin (10 µM). B. Percentage of ASC specks at 10 different images taken for each condition in three independent experiments. C. Western blot of crosslinked cytosolic pellets from PMA-differentiated THP-1 ASC GFP cells stimulated with LPS and nigericin and treated with MM01 and VX-765 at the above-described conditions.

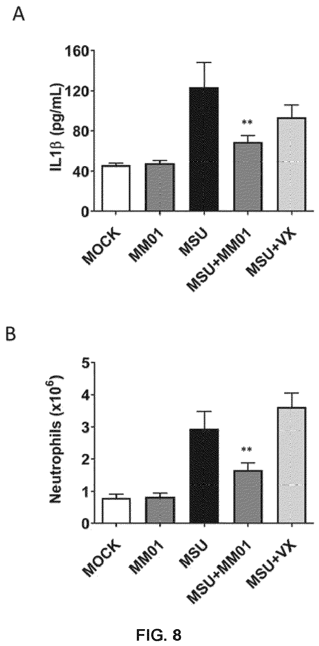

**Fig. 8****.** MM01 inhibits NLRP3 activation in MSU-induced peritonitis. A. ELISA of IL-1β in the peritoneal cavity of C57BL/6 mice intraperitoneally injected with MSU crystals (2 mg/mouse) with or without MM01 (10 mg/kg) or VX-765 (10 mg/kg) treatment. Data are representative of two independent experiments (mean and SD of n=12). B. Neutrophil numbers in the peritoneal cavity of C57BL/6 mice treated in the above-described conditions. Asterisks represent significant differences to the MSU treated control as determined by a one-way ANOVA test with Tukey's multiple post-test comparisons **p<0.05.

### Examples

In order to illustrate the present invention, the inventors provide some examples to show the effect and use of the compounds within the scope of formula (I).

### Example 1: Evaluation of the inflammasome inhibition effect of compounds within the formula (I) of the present invention

The compounds within the formula (I) that has been tested are: MMO1, 2, 3 y 4. Compounds MMO1, 2, 3 y 4, represented in Table 1 below, were acquired in Sigma Aldrich.

The materials and methods employed in these experiments are now described.

### Purification of Procaspase-1 (PC1)

Human His-tagged PC1 was cloned from pET21b-PC1 (Addgene, #11809) in the pFastBac vector for Baculovirus expression (Gibco). The expression plasmid was transformed into DH10Bac E. coli cells (Gibco). Recombinant bacmids were purified and used to transfect Sf9 insect cells (American Type Culture Collection, ATCC). The virus stock was amplified and used to infect suspension cultures. Briefly, Sf9 cells were grown in 2 L of Grace's Insect Medium (Gibco) and infected at 1.5x10⁶cells/mL with a recombinant virus that contained His-tagged PC1 at a multiplicity of infection of 1 (MOI 1). Then they were cultured for 16 h at 27°C with constant gentle agitation to express the protein. Cells were centrifuged at 1000 g for 10 min at 4°C and washed with PBS. The pellet was lysed with buffer lysis (100 mM HEPES-KOH pH 7.5, 50 mM KCI, 7.5 mM MgCl₂, 5 mM NaEDTA, 5 mM NaEGTA and proteases inhibitors: Pepstatin, Leupeptin, and PMSF) using a Douncer and clarified by centrifugation (10000 g, 1 h at 4°C). The resulting supernatant that contained the recombinant protein was purified in a Ni-NTA (Ni2 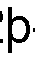 -nitrilotriacetate)-agarose column. After elution, PC1 was further purified by ion-exchange chromatography (Mono Q) in 20 mM of HEPES-KOH pH 7.5, 10 mM KCI, 1.5 mM MgCI2, 1 mM EDTA, 1 mM EGTA.

### Purification of the apoptosis-associated speck-like protein (ASC)

ASC was previously cloned in the pET28a vector (Novagen) for its expression in E. coli cells. Briefly, 8 L of LB medium, plus antibiotics chloramphenicol and kanamycin, were inoculated with an overnight culture of freshly transformed His tagged-ASC-pET28a-BL21 (DE3) pLys E. coli cells and grown at 37°C until OD 0.7. The ASC expression was induced with 0.7 mM IPTG at 28°C for 4 h. Cells were harvested (15 min, 6000 rpm at 4°C), lysed in 300 mL of Buffer A (50 mM NaH2PO4, 300 mM NaCl, pH 8.0) and sonicated. After centrifugation at 11000 rpm for 1 h at 4°C, ASC was present in the inclusion bodies (pellet). Pellets were then resuspended in 100 mL of denaturing Buffer B (50 mM NaH₂PO₄, 300 mM NaCl, 8 M urea, pH 8.0) and resonicated. Under these conditions, the His tagged-ASC protein was solubilized and purified using TALON resin. Re-folding was performed in the TALON resin at a gradient of decreasing urea concentrations, and the protein was finally eluted in 15 mL of Buffer C (50 mM NaH₂PO₄ pH 8.0, 300 mM NaCl, 500 mM imidazole). Desalting was performed in a PD-10 desalting column, and the protein was finally concentrated in a 10 K Amicon concentrator unit.

### Primary screening: the ASC-mediated PC1 reconstitution in vitro assay

Recombinant PC1 (50 nM) was preincubated with the indicated concentrations of compounds for 20 min at RT in Assay Buffer (20 mM HEPES pH 7.5, 10 mM KCI, 1.5 mM MgCl₂, 1mM EDTA, 1 mM EGTA, 1 mM DTT). Then 300 nM of ASC were added and incubated for another 10 min period. Caspase-1 activation was measured using fluorogenic substrate Ac-WEHD-AFC (80 µM; Enzo Life Sciences) by continuously monitoring the release of the AFC (7-Amino-4-trifluoromethylcoumarin) product in a Wallac Victor workstation. The compounds were initially tested at 20 µM with 1% of DMSO. Data are reported as the percentage of inhibition compared with the absence of the compound.

### Secondary screening: the caspase-1 in vitro activation assay

Recombinant caspase-1 (50 nM) was incubated with the indicated concentrations of compounds for 20 min at RT in Assay Buffer. Then fluorogenic substrate Ac-WEHD-AFC (80 µM) was added, and activity was monitored in a Wallac Victor workstation. Data are reported as the percentage of inhibition compared with the absence of the inhibitor.

IC50 values, percentage of C1 inhibition, and drug-likeness measured by OSIRIS Property Explorer are shown in the following table (Table 1):

**Table 1:**

| Compound | IC50(µm) | %INH C1 | OSIRIS |
|---|---|---|---|
| 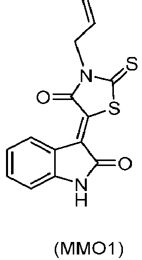 | 0.5±0.1 | 17.0±15.0 | 0.84 |
| 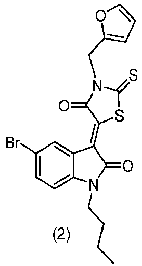 | 0.3±0.1 | 12.0±12.0 | 0.42 |
| 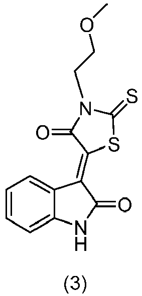 | 0,4±0,2 | 14±13 | 0.79 |
| 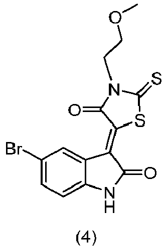 | 1.5±0.8 | 2.0±4.0 | 0.77 |

### Example 2: MM01 blocks ASC mediated PC1 activation in vitro assays.

The inhibitor with the best activity and pharmacologic profile was MM01, which had an IC₅₀ in the nanomolar range (Figure 1B). The inhibition of procaspase-1 activation was also corroborated by the decrease in the formation of p20 subunit by immunoblotting (Figure 1C). Moreover, while the caspase inhibitor zVAD that binds to the active site, inhibits preactivated caspase-1, MM01 do not act on caspase-1 (Figure 1E) nor other apoptotic caspases such as caspase-3 or caspase-9 (Figure 2A and 2B). The apoptosome is a similar complex that cell use to activate procaspase-9 in a cellular apoptotic context. This complex shares several structural characteristics with the inflammasome, it is a molecular platform for the activation of caspases through CARD-CARD interactions. No inhibition of the apoptosome was observed in the presence of MMO1 (Figure 2C), suggesting a new mechanism of action specific for the complex ASC-caspase-1 interaction. Thus, MM01 inhibits specifically ASC-mediated PC-1 activation in an in vitro context.

The materials and methods employed in these experiments are now described. Dose response curve for MM01 compound was performed in the ASC-mediated PC1 reconstitution in vitro assay described in Example 1.

### Caspase-3 activation assay

Compounds were incubated with caspase-3 (2 nM) in reaction buffer (100 mM NaCl, 50 mM Hepes pH 7.4, 10 mM DTT, 1 mM EDTA, 10% glycerol, 0.1% CHAPS) for 20 min at RT. Enzyme activity was spectrophotometrically monitored by adding Ac-DEVD-pNA (200 µM) substrate.

Caspase-9 was activated with kosmotropic salts, in particular with Na-citrate. Briefly, 5 µM caspase-9 was pre-activated for 20 min in SC buffer (50 mM Na2HPO4, 150 mM NaCl, 1.5% sucrose, 0.05% CHAPS, 10 mM DTT, 0.7 M Na-citrate, pH 7.4) at RT. Once pre-activated, the complete reaction was diluted to 100 nM caspase-9 in assay buffer without Na-Citrate and incubated 20 min at RT in the presence or absence of compounds. Enzyme activity was monitored by adding the Ac-LEHD-afc substrate (40 µM) using a Victor 2 spectrofluorimeter.

### Apoptosome activity assay

Apoptosome was reconstituted by incubating rApaf-1 (100 nM) with vehicle (DMSO) or compounds in assay buffer (20 mM HEPES, 10 mMKCI, 1.5 mM MgCI2, 1 mM EDTA, 1 mM EGTA, 1 mM DTT, 0.1 mM PMSF) at 30°C for 30 min. Next, 100 µM dATP and 100 nM Cytc were added and incubated for additional 40 min. Then, caspase-9 (100 nM) was added, and activity was monitored by the Ac-LEHD-afc substrate (50 µM) using a Victor 2 spectrofluorimeter.

### Example 3: MM01 inhibits inflammation signaling mediated by ASC.

Next the activity of these drugs in different cellular models was investigated. Activation of inflammasome induces caspase-1 activation, pro-IL-1β processing and secretion, as well as pyroptosis that is monitored by the release of lactate dehydrogenase from the cell. Then, we assayed the anti-inflammatory action of the compound in THP-1 cells upon NLRP3 inflammasome activation and compared to the specific caspase-1 inhibitor VX-765. ATP-induced NLRP3 activation was inhibited upon treatment with MM01 as evidenced by the decrease of IL-1β and IL-18 secretion (Figure 3A and 3B) and caspase-1 and IL-1β processing (Figure 3C). Interestingly, while no recovery of cell viability was observed upon treatment with VX-765, treatments with MM01 significantively decreased LDH release (Figure 3D), protecting monocytes from inflammatory cell death. In order to confirm this observation, other specific NLRP3 activating stimuli such as nigericin was also tested in PMA-differentiated human THP-1. Once again, MM01 decreased IL-1β and IL-18 secretion and also, pyroptotic cell death (Figure 4). This pyroptosis inhibition is extremely valuable to avoid inflammation spread.

The materials and methods employed in these experiments are now described.

### The inflammasome activation cellular assays

The MM01 compound was evaluated in the THP-1, or PMA-differentiated THP-1 cells (50 nM PMA for 24h) stimulated with lipopolysaccharide (LPS) plus adenosine triphosphate (ATP) to stimulate ASC-dependent inflammasomes NLRP3. Briefly, 1x10⁶ cells were seeded in 6-well plates in 1 mL RPMI media that contained 1% FBS. Cells were either mock-treated or primed with compounds at the indicated concentrations for 30 min, followed by treatment with 100 ng/mL LPS (Sigma) and 2.5 mM ATP (Sigma) for 6 h at 37°C. In the case of nigericin stimulation, cells were primed with LPS for 3h, and then 10 µM nigericin (Sigma) was added for 30 min. Supernatants were harvested and clarified by centrifugation at 1500 rpm at RT, and a cytokine analysis was performed. Both IL-1β secretion and the release of IL-18were monitored by an ELISA assay (BD OptEIA™ Human IL-1β ELISA Kit and Human IL-18 Module Set eBioscience) following the manufacturer's instructions. Cell viability was analyzed in parallel by evaluating the release of lactate dehydrogenase (LDH) according to a commercial kit (CytoTox-ONE™ Homogeneous Membrane Integrity Assay; Promega). The release of LDH was calculated using the formula: the release of LDH (%) = 100x (Abs490 treated -Abs490 untreated cells)/ Abs490 untreated cells lysed with Triton 9% (maximum release of LDH).

### Immunoblotting

The supernatants of the treated cells were precipitated by the chloroform-methanol method. Pellets were obtained by lysing cells in 25 mM of Tris-HCI pH 7.4, 1 mM EDTA, 1 mM EGTA and 1% SDS, plus protease and phosphatase inhibitors. The protein concentration was determined by the BCA protein assay. Samples were separated in a 14% SDS-PAGE gel, transferred to a nitrocellulose membrane and blocked with 5% skimmed milk for 1 h. Then the membrane was incubated overnight with primary antibodies: α-casp1 (1:1000; Cell Signaling 2225), α-IL-1β (1:1000; Acris R1130P), α-NLRP3 (1:1000; Cell Signaling 15101), α-tubulin (1:3000; Abcam ab6160) at 4°C. Membranes were washed and probed with the appropriate secondary antibody conjugated with peroxidase for enhanced chemiluminescence detection (Amersham Pharmacia Biotech).

### Example 4: MM01 inhibits inflamamsome activation in murine and human peritoneal macrophages.

NLRP3 inhibition in peritoneal murine macrophages was evaluated at three different MM01 concentrations. A significant inhibition of IL-1β and subsequently IL-6 release were detected at 5 and 10 µM of MM01 (Figure 5A and 5B) Importantly, this was not correlated with a significant reduction of TNF-α release (Figure 5C) indicating that MM01 action is specific of inflammasome signaling. The release of LDH was also decreased (Figure 5D) as observed in previous cellular models, confirming again that pyroptosis can be blocked by the MM01 treatment.

These evidences were also confirmed in human PBMCs purified from two different blood donors. IL-1β and LDH release were significantly reduced in the human isolated PBMCs stimulated with LPS and ATP. Once again, VX-765 was able to inhibit cytokine release but it did not protect from inflammatory cell death (Figure 5E and 5F). The materials and methods employed in these experiments are now described.

### Murine macrophages and PMBCs cellular assays

Mice (BALB/c) were euthanized, and 5 ml of cold medium was injected (20G needle) into the peritoneal region. The aspirated fluid from peritoneum was centrifuged for 10 min at 400 × g and the cell pellet resuspended in DMEM/F12-10 (1 ml). 40000 cells/well were plated in 24-well plates. Cells were allowed to adhere to the plate by culturing them 2 h at 37°C and then, non-adherent cells were removed by gently washing three times with warm PBS. Macrophages were primed for 4 h with 100 ng/ml pure LPS (Alexis Biochemicals Corporation), in serum-free IMDM medium followed by 30 min with 5 mM ATP. Supernatants were harvested and clarified by centrifugation at 1500 rpm at RT and cytokine, and caspase-1 activation analysis was performed as previously described in Example 3.

Human peripheral blood mononuclear cells (PBMCs) are lymphocytes and monocytes. PBMCs were separated from the whole blood by a density gradient centrifugation method using Ficoll Histopaque. Purified PBMCs were plated in 24-well plates at 5000000 cells/well in free-serum RPMI medium. Cells were primed for 3 h with 10 ng/ml pure LPS followed by 4 h with compounds and 5 mM ATP. Supernatants were harvested and clarified by centrifugation at 1500 rpm at RT and cytokine and caspase-1 activation analysis was performed as previously described.

### Example 5: MM01 spectrum of inflammasome inhibition.

We then evaluated the activity of MM01 on other ASC-dependent and ASC- enhanced inflammasomes. NLRP3, NLRP1 and AIM2 inflammasomes require ASC protein to be functional as it functions as an adaptor protein to connect the sensor protein and caspase-1. In the case of NLRC4 inflammasome, the sensor protein has its own CARD domain and ASC participates in the amplification of the signal.

Treatment of THP-1 cells with LPS and MDP has been previously demonstrated to activate human NLRP1 inflammasome. Pretreatment of cells with MM01 avoided procaspase-1 activation, IL-1β and IL-18 release and protected cells from pyroptotic cell death (Figure 6A and 6B).

The stimulation of AIM2 inflammasome, triggered by cytosolic double-stranded DNA, significantly decreased IL-1β secretion upon MM01 treatment in PMA-differentiated THP-1 cells. Despite this, we were not able to detect any cell death on the stimulated cells (Figure 6C and 6D). NLRC4 inflammasome was first identified because murine macrophages lacking NLRC4 failed to activate caspase-1 after exposure to *Salmonella typhimurium.* The CARD domain of NLRC4 can interact with the CARD domain of PC1, allowing NLRC4 to directly activate the protease. However, caspase-1 cleavage and IL-1β maturation is strongly enhanced in the presence of ASC, activated NLRC4 associates with ASC and co-localizes with the ASC-containing speck after activation by Salmonella Indeed, MM01 impaired cytokine maturation and release on Salmonella infection-induced NLRC4 and in consequence, a decrease in pyroptosis was also observed (Figure 6E and 6F).

The materials and methods employed in these experiments are now described. The molecules were evaluated in the THP-1, or PMA-differentiated THP-1 cells (50 nM PMA for 24h) as described in Example 3. To stimulate NLRP1 inflammasome, cells were either mock-treated or primed with compounds at the indicated concentrations for 30 min, followed by treatment with 100 ng/mL LPS (Sigma) and 50 µg/ml MDP (Sigma) for 6 h at 37°C. In the case of NLRC4 inflammasome induction, cells were pretreated with the compounds for 30 min and then cells were stimulated for 3 h with *Salmonella Typhimurium* (MOI 5). To induce AIM2 inflammasome, cells were pretreated in the same conditions as before and transfected with poly(dA:dT) (200 µg/ml; Sigma) overnight using Lipofectamine 2000 (Invitrogen).
Both IL-1β secretion and the release of IL-18 were monitored as previously described in Example 3.

### Example 6: MM01 inhibits ASC oligomerization.

Oligomerization of ASC can be visualized in immunofluorescent microscopy as specks. A 30 % of LPS-primed THP-1 cells expressing GFP fused to ASC and treated with nigericin had ASC specks (Figure 7A and 7B). As expected, speck formation was not inhibited by VX-765 since caspase activation occurs downstream of inflammasome assembly. Interestingly, MM01 inhibited ASC speck formation (Figure 7A and 7B). In order to confirm this observation, pellets from the LPS plus nigericin-treated cells were chemically crosslinked and ASC oligomers isolated from control cells, stimulated and compound-treated cells. MM01 and not VX-765, inhibited ASC oligomerization,suggesting that MM01 interferes with the protein-protein interaction interphase between ASC and procaspase-1 (Figure 7C).

The materials and methods employed in these experiments are now described.

### ASC speck assay

THP1-ASC-GFP cells were seeded at 1×10⁶/ml the day prior to use in experiments on 35 mm glass-bottom culture dishes. The following day medium was replaced containing the indicated inhibitors or vehicle for 30 min. Cells were then primed with 100 ng/ml LPS for 3h and stimulated with 10 µM nigericin for 30 min. Samples were fixed in 4% paraformaldehyde (PFA) for 10 min at RT and washed several times. Coverslips were prepared in mounting medium plus DAPI and analyzed in a Leica SP8 confocal microscope.

### ASC oligomerization assay

PMA-differentiated THP-1 cells were seeded at 1x10⁶ cells/ml in 6-well plates. Cells were treated with compounds for 30 min, primed with 100 ng/ml LPS for 3h and then stimulated with 10 µM nigericin for 30 min. The supernatants were removed, cells were rinsed in ice-cold PBS and lysed in NP-40 buffer (20 mM HEPES-KOH pH 7.5, 150 mM KCL, 1% NP-40 plus protease inhibitors). Lysates were centrifuged at 330 × g for 10 min at 4 °C. The pellets were washed and resuspended in PBS plus 2 mM disuccinimidyl suberate (DSS) and incubated at RT for 30 min with rotation. Samples were then centrifuged at 330 × g for 10 min at 4 °C. The supernatant was removed, and the cross-linked pellets were resuspended in sample buffer, boiled and analyzed by immunoblotting.

### Example 7: MM01 inhibits NLRP3 activation in vivo.

We next evaluated the efficacy of MM01 in a murine MSU-induced peritonitis model. (Spalinger MR et al. Bio-protocol 2018; 8(5): e2754). Mono-sodium urate (MSU; InvivoGen) crystals are potent activators of the NLRP3 receptor. Upon activation, NLRP3 induces the formation of inflammasome complex, which lead to the production and release of mature IL-1β. In the early phase of peritonitis, the number of infiltrating neutrophils correlates with the production of mature IL-1β. MM01 was injected intraperitoneal 30 min before MSU crystals i.p. injection. MM01 treatment in vivo efficiently suppressed MSU injection-induced IL-1β production and neutrophil recruitment (Figure 8).

The materials and methods employed in these experiments are now described.

### MSU-induced peritonitis mouse model

In vivo model was performed according to the protocol approved by the Institutional Animal Ethical Committee of CIPF. C57BL/6 mice were injected i.p. with 10 mg/kg MM01 or vehicle 30 min before i.p. injection of MSU (2 mg MSU crystals in 200 µl sterile PBS). After 6 h, mice were sacrificed, and peritoneal lavage with 5 ml of PBS was performed. Cytokine IL-1β secretion was determined by ELISA (#DY401 R&D Systems) and neutrophil content measured in ACVLAB Laboratorio Valencia.

### DISCUSSION

In this invention, a new kind of inflammasome inhibitor with an IC50 within the nanomolar range has been described. MM01 exerts an inhibitory effect on PC1 activation *in vitro*, cell-based assays and in a peritonitis induced mouse model. A new point of intervention has been identified, protein-protein interaction between PC1 and ASC is defined as a new challenging surface to modulate. This inhibitor is predicted to act on an allosteric site of PC1 that impedes recruitment to the ASC filament, avoiding ASC specks and activation. Modulation at this level causes efficient inhibition of caspase-1 and cytokine release. Processing of other caspases should be analyzed in order to investigate how ASC oligomers can be contributing to activate other inflammatory caspases.

MM01 is also able to significantly reduce the release of LDH caused by inflammasome activation in THP-1 cells. Compound VX-765 is the most effective inhibitor of caspase-1 of the state of the art as it acts on the catalytic site of the enzyme (Stack JH et al. J Immunol. 2005 August 15;175 (4): 2630-2634), but does not decrease pyroptosis in THP-1 cells. MM01 is able to inhibit pyroptosis and enhance cell recovery. This result supports the hypothesis that targeting ASC-mediated PC1 activation is an advantage for cell protection over active caspase-1 inhibition. In line with the data, recent research has given rise to the development of a single domain antibody fragment that specifically recognizes the CARD domain of human ASC (Schmidt FI et al. J Exp Med. 2016 May 2;213(5):771-90). This antibody impairs ASC (CARD) interactions, inhibits inflammasome activation and, more importantly, protects cells from inflammatory cell death. The results provides herein reinforce the idea that the perturbation of ASC interaction interfaces could be a potential target for the development of broad-spectrum anti-inflammatory agents with improved cell recovery capabilities.

So far, the available clinical treatment in several inflammatory human diseases is the use of drugs that target IL-1β as antibodies (canakinumab) or recombinant IL-1β receptor antagonist (anakinra). These treatments although effective have a dark face, they produce undesirable immunosuppressive effects and at the same time, they do not cover all the symptoms as other proinflammatory cytokine and pyroptotic cell death participate in the physiopathology of the diseases. In addition, biological agents are expensive and small-molecule compounds are in general more cost effective. Acting upstream on the signaling it may prevent the contribution of IL-18 and pyroptosis to the pathology.

In the state of the art, a few small-molecule compounds have shown anti-inflammatory effects on NLRP3 inflammasome activation *in vitro*, most of them have low efficacy or unspecific effects. The most interesting are MCC950 (Coll RC et al. Nat Med. 2015 Mar;21(3):248-55) and CY-09 (Jiang H et al. J Exp Med. 2017 Nov 6;214(11):3219-3238). The first one has a strong inhibitory activity and is efficient in several mice models of NRLP3-related diseases,but the mechanism is still under discussion. The second one, CY-09 specifically inhibits NLRP3 inflammasome activation in vitro and in vivo by blocking its ATPase activity. However, more potent analogues and further studies analyzing cross-reactivity against the enzymatic activity of protein kinases and ATPases would be needed/recommended.
It may be true that acting selectively over one inflammasome can have less side effects and in particular, be less immunosuppressive or impair less the host defense.

## Claims

1. A compound of formula (I), 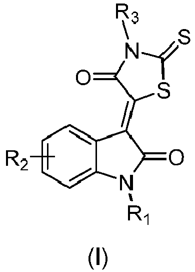
a pharmaceutically acceptable salt, isomer or a solvate thereof, wherein
R₁ is selected form the list comprising: H, optionally substituted (C₁-C₄) alkyl and optionally substituted (C₁-C₄) alkenyl,
R₂ is H or halogen,
R₃ is an optionally substituted (C₁-C₄) alkyl or optionally substituted (C₁-C₄) alkenyl, for use as inflammasome inhibitor.

2. Compound for use, according to claim 1, in the treatment and/or prevention of inflammasome-related diseases.

3. Compound for use, according to any of claims 1 to 2, wherein R₁ is H.

4. Compound for use, according to any of claims 1 to 3, wherein R₂ is H.

5. Compound for use, according to any of claims 1 to 4, wherein R₃ is an optionally substituted (C₁-C₄) alkenyl group.

6. Compound for use, according to any of claims 1 to 5, wherein R₃ is an optionally substituted C₃ alkenyl group.

7. Compound for use, according to any of claims 1 to 4, wherein R₃ is an optionally substituted (C₁-C₄) alkyl group.

8. Compound for use, according to claim 7, wherein R₃ is (C₁-C₄) alkyl group substituted by a -O(C₁-C₃)alkyl group or by a heterocycle.

9. Compound for use, according to claim 1, wherein the compound is selected from the next list:

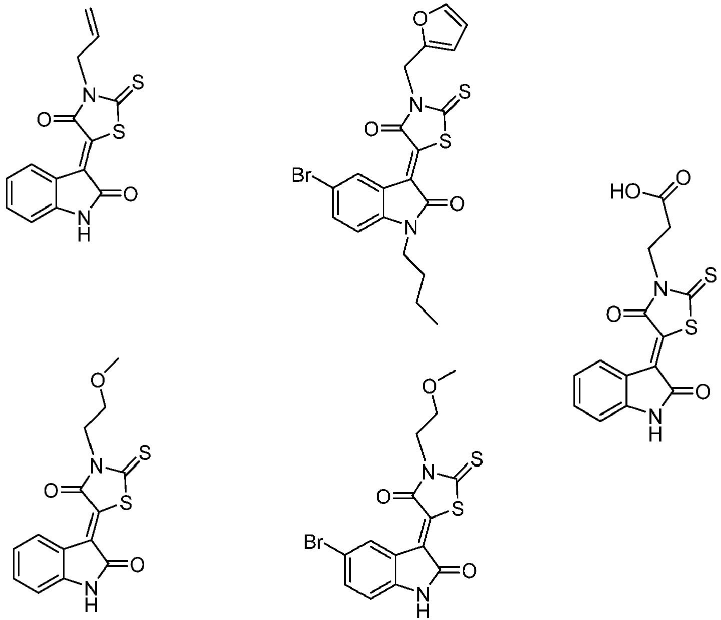

10. A pharmaceutical composition comprising the compound of formula (I), as described in any of claims 1 to 9, a pharmaceutically acceptable salt or a solvate thereof, for use in the treatment and/or prevention of inflammasome-related diseases.

11. A compound for use according to claims 1 to 9 or a pharmaceutical composition for use, according to claim 8, wherein the inflammasome-related disease is a disease selected from: monogenic inflammasomopathie; infectious disease, inflammatory disease neurodegenerative pathology or autoimmune disease.

12. A compound or a pharmaceutical composition for use, according to claim 11, wherein the inflammasome-related disease is selected from the list comprising: Cryopyrin-Associated Periodic Syndromes (CAPS), Familial Mediterranean Fever (FMF), Schnitzler Syndrome, Hyper-lgD Syndrome (HIDS), PAPA Syndrome, Deficiency of IL-1 Receptor Antagonist (DIRA), Zika infection, HIV infection, West Nile virus infection, meningitis, Lupus erythematosus, topic and contact dermatitis, acne, hidradenitis suppurative, psoriasis, vitiligo, Crohn's disease (CD),ulcerative colitis (UC), dysbiosis, multiple sclerosis, amyotrophic lateral sclerosis, prion diseases, Parkinson's disease, Alzheimer's disease, Cancer, peritonitis, gout, diabetes, rheumatoid arthritis, atherosclerosis, Chronic obstructive pulmonary disease (COPD), hypertension, asthma, allergies, ischemic stroke, ischemia-reperfusion injury and Alopecia areata.

13. Use a compound of formula (I) as described in any of the previous claims 1 to 9, a pharmaceutically acceptable salt or a solvate thereof, as a biotechnology tool for the study of the implication of the inflammasome in biological processes.
